## Europäisches Patentamt
## European Patent Office
## Office européen des brevets

(11) Veröffentlichungsnummer: **0 058 753**
**B1**

(12) **EUROPÄISCHE PATENTSCHRIFT**

(45) Veröffentlichungstag der Patentschrift:
10.07.85

(51) Int. Cl.⁴: **A 61 F 2/34**

(21) Anmeldenummer: 81108627.1

(22) Anmeldetag: 21.10.81

(54) Im Becken verankerbare Gelenkpfanne.

(30) Priorität: 20.02.81 CH 1133/81

(43) Veröffentlichungstag der Anmeldung:
01.09.82 Patentblatt 82/35

(45) Bekanntmachung des Hinweises auf die Patenterteilung:
10.07.85 Patentblatt 85/28

(84) Benannte Vertragsstaaten:
AT DE FR GB IT NL

(56) Entgegenhaltungen:
EP - A - 0 038 903
DE - A - 2 857 297
FR - A - 2 225 924
FR - A - 2 301 217
FR - A - 2 380 021

(73) Patentinhaber: GEBRÜDER SULZER
AKTIENGESELLSCHAFT, Zürcherstrasse 9,
CH-8401 Winterthur (CH)
Patentinhaber: Protek AG, Stadtbachstrasse 64,
CH-3001 Bern (CH)

(72) Erfinder: Niederer, Peter Gino, Dipl.-Ing. ETH,
Reichenbachstrasse 6, CH-3052 Zollikofen (CH)
Erfinder: Frey, Otto, Walrütistrasse 56,
CH-8400 Winterthur (CH)

(74) Vertreter: Dipl.-Ing. H. Marsch Dipl.-Ing. K. Sparing
Dipl.-Phys.Dr. W.H. Röhl Patentanwälte,
Rethelstrasse 123, D-4000 Düsseldorf (DE)

**Beschreibung**

Die Erfindung betrifft eine im Becken verankerbare Gelenkpfanne zur Aufnahme eines kugeligen Gelenkkopfes einer Hüftgelenkprothese in einem rotationssymmetrischen Pfannenhohlkörper, wobei der im Becken zu verankernde Pfannenkörper ein sich im Querschnitt an seiner Außenseite verjüngender Körper ist, in dessen den größeren Querschnitt aufweisendem Ende der Pfannenhohlraum angeordnet ist und der über den Umfang verteilt und entlang einer Mantellinie verlaufend, hervorspringende Verankerungselemente aufweist, die durch im wesentlichen quer zur Rotationsachse des Pfannenhohlraumes ausgerichtete Einschnitte in Querrippen unterteilt sind.

Eine Gelenkpfanne der vorstehend genannten Art ist aus der FR-A-2 380 021 bekannt; bei dieser Konstruktion bestehen die Verankerungselemente aus zirkularen, aus der kugelschalenförmigen Außenfläche hervorstehenden Rippen, die durch mehrere, zu ihnen senkrechte gleichmäßig über den Umfang verteilte Nuten unterteilt sind. Diese Pfanne ist ausschließlich für einen Einsatz in ein Knochenzementbett bestimmt und für eine zementfreie Verankerung nicht geeignet.

Weiterhin ist bereits eine Gelenkpfanne vorgeschlagen worden (EP-A-0 038 903), bei der als Verankerungselemente aus dem Mantel des Pfannengrundkörpers hervorspringende Halbzylinder vorgesehen sind, die mit Querrippen versehen sind.

Aufgabe der vorliegenden Erfindung ist es, bei zementfreier Verankerung die Haftfestigkeit einer Gelenkpfanne, deren Grundkörper ähnlich demjenigen der unmittelbar vorstehend beschriebenen Pfanne gestaltet ist, zu verbessern. Erfindungsgemäß wird diese Aufgabe dadurch gelöst, daß alle Verankerungselemente die gleiche Form aufweisen, daß weiterhin alle Querrippen in Aufsicht auf die Flächen der Einschnitte an der von der Rotationsachse fern liegenden Seite kreisbogenförmig gerundet sind, wobei die Länge von mit einem Ende an die Mantelfläche des Pfannenkörpers angesetzten, zu der kreisbogenförmigen Seite führenden Rechteckseiten innerhalb einer Gesamtheit von ein Verankerungselement bildenden Querrippen von Null bei der am breitesten Ende des sich verjüngenden Körpers angeordnete Querrippe bis zu einem Maximalwert bei der am entgegengesetzten Ende sich verjüngenden Körpers angeordneten Querrippe zunimmt, und daß ferner der Ausschnitt aus dem Kreisbogen der kreisbogenförmig gerundeten Seite in gleicher Richtung von einem Minimalwert bis zu einem Maximalwert eines Halbkreisbogens zunimmt, und daß schließlich mindestens ein Teil der Querrippen elastisch ausgebildet ist.

Beim Einsetzen der neuen Pfanne in ein operativ an ihre Form und Abmessungen angepaßtes Lager im Beckenknochen verbiegen sich mindestens eine Anzahl der elastischen Querrippen, die entgegen der Verjüngung und der Einsetzrichtung des Pfannenkörpers größer werden, gegen die Einsetzrichtung und bilden so eine Vielzahl von Widerlagern gegen eine Herausnahme oder ein Herausarbeiten des Pfannenkörpers. Die gegenläufigen Verjüngungen von Verankerungselementen verhindern dabei zusätzliche Bewegungen in und gegen die Einsetzrichtung, womit die Gefahr von Pfannenlockerungen erheblich vermindert wird.

Die operative Anpassung des Lagers im Bekkenknochen kann dabei beispielsweise so erfolgen, daß zunächst möglichst exakt ein Hohlraum entsprechend der Außenseite des Pfannengrundkörpers aus dem Knochen gefräst wird; in einem zweiten Arbeitsgang werden dann mit Hilfe eines Bohrinstruments, das relativ zur gefrästen Ausnehmung in einer bestimmten Lage fixiert wird, die Hohlräume für die entgegen dem Grundkörper größer werdenden Verankerungselemente, ebenfalls möglichst genau in die Ausfräsung gebohrt. Dabei hat es sich für die genaue Zentrierung des Bohrinstruments als vorteilhaft erwiesen, wenn der Pfannenkörper eine exzentrische Markierung zur Festlegung einer eindeutigen Einbaulage hat.

Der Winkel, den die die Scheitelpunkte aller kreisförmig gerundeten Querrippenseiten eines Verankerungselementes verbindende Scheitellinie mit der Rotationsachse des Pfannenhohlraumes bilden, ist materialabhängig und beispielsweise unter anderem wesentlich von der Elastizität des Pfannenmaterials beeinflußt. Experimentell sind dabei für diesen Winkel Werte von 1° — 20° Neigung gegen die Rotationsachse als besonders geeignet ermittelt worden.

Der für die erfindungsgemäßen Pfannen bevorzugte Werkstoff ist hochmolekulares Polyäthylen der Klassifikationen HDPE und UHMW; für dieses Material sind Winkel von 5° — 15° zwischen der Scheitellinie und der Rotationsachse besonders vorteilhaft. Weiterhin ergibt sich bei diesem Material ein besonders fester Sitz im Beckenknochen, wenn die Dicke der Querrippen zwischen 0,3 und 1,5 mm und ihr Abstand zwischen 0,5 und 2,5 mm sind.

Im folgenden wird die Erfindung anhand eines Ausführungsbeispiels im Zusammenhang mit der Zeichnung näher erläutert.

Fig. 1 zeigt eine Seitenansicht eines Ausführungsbeispiels der neuen Gelenkpfanne, das mit einen unsymmetrischen Rand für den eigentlichen hohlkugelförmigen Pfannenhohlraum versehen ist;

Fig. 2 ist eine Aufsicht auf die Gelenkpfanne in Richtung der Rotationsachse ihres Pfannenhohlraumes.

Der Pfannenkörper 1 der Gelenkpfanne ist bei einem gezeigten Beispiel im Grundkonzept ein rotationssymmetrischer, konischer Kegelstumpf, dessen Symmetrieachse mit 2 bezeichnet ist; die Symmetrieachse 2 ist auch gleichzeitig die Rotationsachse für den den Pfannenhohlraum 10 er

zeugenden Kreisbogen.

Aus der Mantelfläche des Pfannengrundkörpers 1 springen entlang von Mantellinien 3 des Grundkörpers 1 verlaufende Verankerungselemente 4 hervor, die gleichmäßig über den Umfang verteilt sind und bezüglich der Symmetrieachse 2 eine 6-zählige Symmetrie aufweisen; die einzelnen Verankerungselemente 4 weisen untereinander alle die gleiche Form auf. Erfindungsgemäß erweitern sich die Verankerungselemente 4 gegenläufig zur konischen Verjüngung des Pfannenkörpers 1.

Quer zur Rotationsachse 2 sind die Verankerungselemente 4 mit Einschnitten versehen, so daß sie in lappenartige Querrippen 5 unterteilt sind, die in Aufsicht auf die Flächen der Einschnitte an der von der Rotationsachse fern liegenden Seite kreisbogenförmig gerundet sind. Die Länge und damit die Elastizität der Querrippen 5 wird in Richtung der Erweiterung der Verankerungselemente 4 größer, was dadurch erreicht wird, daß die Länge von — die gerundeten Seiten mit der Mantelfläche des Pfannenkörpers 1 verbindenden — Rechteckseiten innerhalb eines Verankerungselementes 4 von null — bei der am breitesten Ende des Pfannenkörpers 1 angeordneten Querrippe 5 — bis zu einem Maximalwert am entgegengesetzten Ende des Pfannenkörpers 1 zunimmt. Die Zwischenräume zwischen den Querrippen 5 dienen zusätzlich der Vergrößerung der Verankerungsoberfläche und dem Einwachsen von spongiosem Knochengewebe.

Der Neigungswinkel $\alpha$, den die — alle Scheitelpunkte der Querrippen 5 eines Verankerungselementes 4 verbindende — Scheitellinie der kreisbogenförmigen Querrippenseiten mit der Rotations- oder Symmetrieachse 2 bildet, beträgt im vorliegenden Beispiel eines Pfannenkörpers aus Polyäthylen etwa 10°. Je nach Elastizität des Pfannenwerkstoffes kann er auch andere Werte zwischen 1° und 20° annehmen, wobei sein günstigster Wert für jedes Material experimentell ermittelt wird.

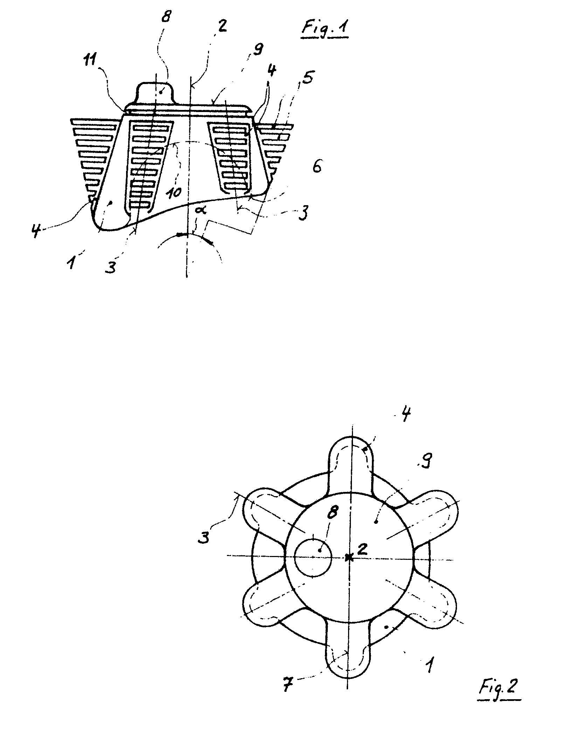

Der den hohlkugelförmigen Pfannenhohlraum 10 begrenzende Rand 6 der Gelenkpfanne ist, wie Fig. 1 erkennen läßt, unsymmetrisch ausgebildet, d. h. bezüglich einer Mittelebene 7 (Fig. 2) durch die Symmetrieachse 2 auf einer Seite weiter heruntergezogen als auf der anderen. Dadurch wird es notwendig, daß die Gelenkpfanne eine bestimmte Einbaulage im Beckenknochen einnimmt. Da ihr Pfannenkörper 1, einschließlich der Verankerungselemente 4, jedoch rotationssymmetrisch aufgebaut ist, ist als Markierung für die Festlegung dieser Einbaulage ein exzentrisch angeordneter Zapfen 8 auf der oberen Basisfläche 9 des kegelstumpfförmigen Pfannenkörpers 1 vorgesehen; der Zapfen 8 bzw. die dafür notwendige Ausnehmung im Beckenknochen dient gleichzeitig als Fixpunkt für die erwähnte Zentrierung eines Bohrinstruments zur Herstellung der die Verankerungselemente 4 aufnehmenden Bohrungen.

Bei der gezeigten Ausführungsform enden die Verankerungselemente 4 unterhalb der Niveaufläche 9 des Pfannenkörpers 1; in seinem wulstfreien Teil kann daher eine Ringnut 11 für die Einlage eines die Pfannenlage im Röntgenbild markierenden Metalldrahtes vorgesehen sein.

Vor dem Einsetzen des Pfannenkörpers 1 wird der Beckenknochen in der bereits beschriebenen Weise operativ entsprechend vorbereitet. Die Außenform des Pfannenkörpers ist nicht auf die Kegelstumpfform beschränkt, sondern kann auch beispielsweise halbkugelartig oder pyramidenstumpfförmig sein.

Das Einsetzen der Pfanne in dem Beckenknochen erfolgt in bekannter Weise so, daß die in den Figuren links dargestellte Hälfte mit dem Zapfen 8 lateral-cranial im Körper angeordnet ist, während die rechte Hälfte in Richtung medial-caudal zu liegen kommt.

## Patentansprüche

1. Im Becken verankerbare Gelenkpfanne zur Aufnahme eines kugeligen Gelenkkopfes einer Hüftgelenksprothese in einem rotationssymmetrischen Pfannenhohlkörper (10), wobei der im Becken zu verankernde Pfannenkörper (1) ein sich im Querschnitt an seiner Außenseite verjüngender Körper ist, in dessen den größeren Querschnitt aufweisendem Ende der Pfannenhohlraum (10) angeordnet ist und der über den Umfang verteilt und entlang einer Mantellinie (3) verlaufend, hervorspringende Verankerungselemente (4) aufweist, die durch im wesentlichen quer zur Rotationsachse (2) des Pfannenhohlraumes (10) ausgerichtete Einschnitte in Querrippen (5) unterteilt sind, dadurch gekennzeichnet, daß alle Verankerungselemente (4) die gleiche Form aufweisen, daß weiterhin alle Querrippen (5) in Aufsicht auf die Flächen der Einschnitte an der von der Rotationsachse (2) fern liegenden Seite kreisbogenförmig gerundet sind, wobei die Länge von mit einem Ende an die Mantelfläche des Pfannenkörpers (1) angesetzten, zu der kreisbogenförmigen Seite führenden Rechteckseiten innerhalb einer Gesamtheit von ein Verankerungselement (4) bildenden Querrippen (5) von Null bei der am breitesten Ende des sich verjüngenden Körpers (1) angeordnete Querrippe (5) bis zu einem Maximalwert bei der am entgegengesetzten Ende sich verjüngenden Körpers (1) angeordneten Querrippe (5) zunimmt, und daß ferner der Ausschnitt aus dem Kreisbogen der kreisbogenförmig gerundeten Seite in gleicher Richtung von einem Minimalwert bis zum Maximalwert eines Halbkreisbogens zunimmt, und daß schließlich mindestens ein Teil der Querrippen (5) elastisch ausgebildet ist.

2. Gelenkpfanne nach Anspruch 1, dadurch gekennzeichnet, daß materialabhängig der Winkel ($\alpha$) der Rotationsachse (2) des Pfannenhohlraumes (10) mit der Scheitellinie, die die Scheitelpunkte aller kreisbogenförmig gerundeten Querrippenseiten eines Verankerungselementes (4) verbindet, zwischen 1° — 20° beträgt.

3. Gelenkpfanne nach Anspruch 1, dadurch gekennzeichnet, daß der Pfannenkörper eine exzentrische Markierung (8) zur Festlegung einer eindeutigen Einbaulage hat.

4. Gelenkpfanne nach Anspruch 2, dadurch gekennzeichnet, daß für Polyäthylen als Pfannenmaterial der Winkel $(\alpha)$ 5° — 15° beträgt.

5. Gelenkpfanne nach Anspruch 4, dadurch gekennzeichnet, daß die Dicke der Querrippen (5) zwischen 0,3 und 1,5 mm und ihr Abstand zwischen 0,5 und 2,5 mm sind.

## Claims

1. An acetabulum anchorable in the pelvis and adapted to receive a ball head of a hip joint prosthesis in a rotationally symmetrical socket (10), the anchorable pelvis member (1) being a member which narrows on its outside, the socket (10) being disposed in that end of the member (1) which has the larger cross-section, the member (1) having projecting anchorage elements (4) which are distributed over the periphery and along a generatrix (3) and which are subdivided by notches into transverse ribs (5), the notches extending substantially tranversely to the rotational axis (2) of the socket (10), characterised in that all the anchorage elements (4) have the same shape, all the transverse ribs (5) are rounded arcuately, as seen in plan looking on to the surfaces of the notches, on the side remote from the rotational axis (2), the length of rectangle sides which have one end on the generated surface of the member (1) and which extend to the arcuate side and which are inside a totality of transverse ribs (5) forming an anchorage element (4) increasing from zero at the transverse rib (5) disposed at the widest end of the narrowing member (1) to a maximum at the transverse rib (5) disposed at the opposite end of the narrowing member (1), and the section of the arc of the arcuate side increases in the same direction from a minimum value to a maximum value of a semicircle, and at least some of the transverse ribs (5) are resilient.

2. An acetabulum according to claim 1, characterised in that, in dependence upon material, the angle $(\alpha)$ formed by the rotational axis (2) of the socket (10) with the line interconnecting the apexes of all the arcuately rounded transverse-rib sides of an anchorage element (4) is from 1° to 20°.

3. A socket according to claim 1, characterised in that the member (10) has an eccentric marking (8) for foolproof location in assembly.

4. An acetabulum according to claim 2, characterised in that the angle $(\alpha)$ is from 5° to 15° when polythene is the socket material.

5. An acetabulum according to claim 4, characterised in that the thickness of the transverse ribs (5) is between 0,3 and 1,5 mm and the spacing between them is between 0,5 and 2,5 mm.

## Revendications

1. Cuvette d'articulation pouvant être ancrée dans le bassing pour recevoir la tête sphérique, d'articulation d'une prothèse coxo-fémorale dans une cavité (10) à symétrie de révolution de la cuvette, le corps (1) de cette cuvette, devant être ancré dans le bassin, étant un corps décroissant de section à sa face externe, présentant la cavité (10) de la cuvette dans son extrémité ayant la plus grande section, et comportant des éléments d'ancrage (4) saillants, répartis sur son pourtour, s'étendant le long d'une génératrice (3) et subdivisés en des nervures transversales (5) par des encoches orientées sensiblement perpendiculairement à l'axe de rotation (2) de la cavité (10) de la cuvette, caractérisée par le fait que tous les éléments d'ancrage (4) présentent la même forme; par le fait que, en outre, toutes les nervures transversales (5) sont arrondies en arc de cercle, lorsque les surfaces des encoches sont observées en plan, du côté éloigné de l'axe de rotation (2), la longueur des côtés d'un rectangle, portant par une extrémité contre la surface périphérique du corps (1) de la cuvette et débouchant du côté configuré en arc de cercle, croissant, au sein d'un ensemble de nervures transversales (5) formant un élément d'ancrage (4), de la valeur zéro sur la nervure transversale (5) située à l'extrémité la plus large du corps (1) de section décroissante, jusqu'à une valeur maximale sur la nervure transversale (5) disposée à l'extrémité opposée du corps (1) de section décroissante; par le fait que l'échancrure de l'arc de cercle cu côté arrondi en arc de cercle croît en outre, dans la même direction, d'une valeur minimale jusqu'à la valeur maximale d'un demi-cercle; et par le fait que, enfin, au moins une partie des nervures transversales (5) est réalisée élastique.

2. Cuvette d'articulation selon la revendication 1, caractérisée par le fait que, en fonction du matériau, l'angle $(\alpha)$ formé par l'axe de rotation (2) de la cavité (10) de la cuvette avec la ligne des sommets, reliant les points des sommets de tous les côtés des nervures transversales d'un élément d'ancrage (4) arrondis en arc de cercle, mesure entre 1° et 20°.

3. Cuvette d'articulation selon la revendication 1, caractérisée par le fait que le corps de cette cuvette présente un repère excentré (8) en vue de la détermination d'une position d'incorporation précise.

4. Cuvette d'articulation selon la revendication 2, caractérisée par le fait que, lorsque le matériau constituant cette cuvette est du polyéthylène, l'angle $(\alpha)$ mesure entre 5° et 15°.

5. Cuvette d'articulation selon la revendication 4, caractérisée par le fait que l'épaisseur des nervures transversales (5) est comprise entre 0,3 et 1,5 mm, leur espacement étant compris entre 0,5 et 2,5 mm.

Fig. 1

Fig. 2